# EUROPÄISCHE PATENTSCHRIFT

(11) **EP 1 631 205 B1**
(45) Veröffentlichungstag und Bekanntmachung des Hinweises auf die Patenterteilung: **04.08.2010**
(21) Anmeldenummer: 03817231.8
(22) Anmeldetag: 12.06.2003
(51) Int. Cl.: A61B 17/72

(54) **CHIRURGISCHER NAGEL**
SURGICAL NAIL
CLOU CHIRURGICAL

(43) Veröffentlichungstag der Anmeldung: 08.03.2006
(73) Patentinhaber: Synthes GmbH, 4436 Oberdorf (CH)
(72) Erfinder: SCHLIENGER, André, CH-4142 Münchenstein (CH); BUETTLER, Markus, CH-4717 Mümliswil (CH); SENN, Peter, CH-4437 Waldenburg (CH); RAEHLE, Christian, CH-4053 Basel (CH)
(74) Vertreter: Lusuardi, Werther
(86) Internationale Anmeldenummer: PCT/CH2003/000375
(87) Internationale Veröffentlichungsnummer: WO 2004/110290

(56) Entgegenhaltungen:
- DE-A- 19 629 011
- US-A- 5 713 901
- US-A1- 2002 151 898
- US-A1- 2002 173 792

## Beschreibung

Die Erfindung betrifft einen chirurgischen Nagel, insbesondere einen intramedullären Marknagel, gemäss dem Oberbegriff des Patentanspruchs 1.

Die Verriegelung von Marknägeln gehört zum Stand der Technik. Die Einführung der Verriegelungsschrauben oder Verriegelungsbolzen (im folgenden wird nur noch der Begriff Verriegelungsschraube verwendet, der aber auch den Begriff Verriegelungsbolzen mitumfassen soll) in die Querbohrungen des Marknagels erfolgt entweder mit Hilfe eines bildgebenden Verfahrens (Röntgenkontrolle) oder einer mehr oder weniger komplizierten Zielvorrichtung. In beiden Fällen ist eine gewisse Zielungenauigkeit nicht zu vermeiden, d.h. die Schraubenspitze lässt sich nicht exakt koaxial zur Mittelachse der Querbohrung ausrichten, sondern weicht davon um einen gewissen Betrag ab. Damit die Verriegelungsschraube trotz dieses Zielfehlers in die Querbohrung mündet und durch diese hindurchgebracht werden kann, wird der Aussendurchmesser der Schraube relativ zum Durchmesser der Querbohrung unterdimensioniert. Bleibt die Zielungenauigkeit im Rahmen dieser Unterdimensionierung, so kann die Verriegelungsschraube trotz des Zielfehlers problemlos durch die Querbohrung geführt werden. Allerdings weist nun die Verriegelungsschraube - wegen der Unterdimensionierung - ein gewisses Spiel auf relativ zur Querbohrung.

Dieses Spiel definiert, um welchen Betrag sich die Knochenhauptfragmente, welche mittels Verriegelungsschrauben im entsprechenden Verriegelungsloch fixiert werden, relativ zum Nagel und somit aufgrund der Starrheit des Nagels auch relativ zu anderen mit demselben Nagel befestigten Knochenhauptfragmente bewegen können. Um die Anwendbarkeit der Verriegelung für den Chirurgen zu garantieren, ist dieses Spiel zwar unumgänglich, doch ist es klinisch bei gewissen Indikationen (z.B. im Falle von metaphysären Fragmenten) unerwünscht.

Selbst Nägel mit vollem Querschnitt, welche im Verriegelungsloch ein Innengewinde aufweisen können, sind nicht spielfrei. Das Innengewinde verhindert lediglich, dass der Nagel sich axial auf der Verriegelungsschraube verschieben kann.

Aus der WO 2004/098424 AESCULAP (Basis für den Oberbegriff des Anspruchs 1) ist ein Marknagel mit einem Fixierkörper zur Aufnahme einer Verriegelungsschraube bekannt.

Aus der US 6,296,645 HOVER ET AL. ist ein hohler, intramedullärer Marknagel aus Metall bekannt, der in den als Fenster bezeichneten sich diametral gegenüberstehenden Mantelöffnungen der Querbohrung einen oder zwei Kunststoffeinsätze aufweist, durch welche die Verriegelungsschraube eingeführt werden kann. Nachteilig bei diesem bekannten Marknagel ist der Umstand, dass die fensterartigen Kunststoffeinsätze unter den im klinischen Einsatz auftretenden Belastungen eingedrückt werden können, so dass die erwünschte Funktion verloren geht. Aber selbst bei einer sehr vorsichtigen Manipulation dürften die beiden Kunststoffeinsätze beim Durchführen der Verriegelungsschraube aus ihrem "Fenster" gedrückt werden, was ebenfalls zu einem Verlust der Funktion führt.

Hier will die Erfindung Abhilfe schaffen. Der Erfindung liegt die Aufgabe zugrunde, einen chirurgischen Nagel, insbesondere einen intramedullären Marknagel zu schaffen, mit dem das vorhandene Spiel zwischen ihm und der Verriegelungsschraube risikolos eliminiert werden und eine verbesserte Haltekraft zwischen Verriegelungsschraube und Marknagel erzielt werden kann, ohne dass der Anwender eine erhöhte Präzision bei seiner Arbeit aufbringen muss.

Die Erfindung löst die gestellte Aufgabe mit einem chirurgischen Nagel, welcher die Merkmale des Anspruchs 1 aufweist.

Damit sind folgende Vorteile erzielbar:
a) die Zielgenauigkeit bei der Einbringung der Verriegelungsschraube ist unbeeinträchtigt;
b) Nagel und Einsatz können separat steril verpackt werden und der Chirurg kann wählen ob er den Nagel ohne Einsatz oder mit Einsatz verwenden will. In letzterem Fall kann der Chirurg den Einsatz selbst in Nagel einführen und gegebenenfalls auch wieder entfernen. Verwendet der Chirurg den Nagel ohne Einsatz so bleibt dieser weiterhin steril verpackt für eine nächste Verwendung. Der Arzt kann somit noch intraoperativ wählen, ob er eine winkelstabile Verriegelung der Verriegelungsschraube einsetzen will oder nicht, wobei der Begriff "winkelstabil" eine Einschränkung gewisser Freiheitsgrade bedeutet.
c) Möglichkeit der winkelstabilen Fixierung des Knochenfragments in gewissen Richtungen für einen bestimmten Betrag der Last.

Eine bevorzugte Weiterbildung der Erfindung besteht darin, dass der Einsatz stabförmig ausgebildet ist und durch die Längsbohrung des Nagels bis in den Bereich der Querbohrungen einführbar ist. Der Chirurg kann den Einsatz auch noch nach erfolgter Implantation des Nagels (ohne Einsatz) einsetzen, indem der Einsatz von proximal in die Längsbohrung bis in den Bereich der Querbohrungen vorschiebt.

Das Material m des Einsatzes weist vorzugsweise eine geringere Druckfestigkeit f_{d} < F_{d} sowie eine geringere Zugfestigkeit f_{z} < F_{z} auf als das Material M auf.

Das Elastizitätsmodul "e" des Einsatzes beträgt vorzugsweise "e" < 0,8 E und typischerweise "e" < 0,7 E .

Bei einer besonderen Ausführungsform besteht das Material m des Einsatzes aus einem biokompatiblen Kunststoff, vorzugsweise einem Polyethylen oder einem hochmolekulares Polyethylen (HMWPE) . Dies hat den Vorteil, dass kein Abbau des Kunststoffs mit unbekannten Abbauprodukten erfolgt.

Bei einer alternativen besteht das in die Längsbohrung des hohlen Nagels eingebrachte Material geringerer Härte aus einem bioresorbierbarem Kunststoff, welcher vorzugsweise ein Polylactid ist. Bei dieser Ausführung resultiert anfänglich eine spielfreie Querverriegelung des Marknagels, welche dann mit zunehmender Resorption des Polymers sukzessive wieder aufgehoben wird, so dass die Querverriegelungsschraube relativ zum Marknagel und somit auch die versorgten Knochenfragmente wieder beweglich werden. Es erfolgt somit eine Dynamisierung der Knochenfragmente nach erfolgter Frakturkonsolidierung.

Ein weiterer Vorteil des bioresorbierbaren Materials besteht darin, dass die beim Hindurchschrauben einer Verriegelungsschraube durch den Nagel entstehenden Späne vom Körper abgebaut werden können.

Bei einer weiteren Ausführungsform besitzt der Nagel mindestens zwei Querbohrungen, vorzugsweise mindestens drei Querbohrungen.

Eine der Querbohrungen kann auch als Langloch mit dem Querschnittsprofil F ausgebildet sein, wobei die längere Dimension "a" des Langlochs in axialer Richtung des Nagels angeordnet ist.

Das Material "m" des Einsatzes weist vorzugsweise auch eine geringere Dichte ρ₁ als das Material M mit der Dichte ρ₂ auf, wobei vorzugsweise ρ₁ < 0,8 ρ₂ ist.

Der Nagel kann eine in die Querbohrung (mit dem Querschnittsprofil F) und durch den Einsatz einführbare Verriegefungsschraube oder einen Verriegelungsbolzen umfassen, dessen Aussengewinde, beziehungsweise dessen gewindeloser Schaft einen Aussendurchmesser "d" aufweist, welcher der Bedingung a > d < b gehorcht.

Der Durchmesser der Längsbohrung des Nagels in Richtung seiner Zentralachse kann variabel gestaltet sein und die Längsbohrung vorzugsweise einen zirkulären Absatz aufweisen.

Bei einer weiteren Ausführungsform kann der stabförmige Einsatz auch eine radial, quer zu seiner Längsachse verlaufende Vertiefung aufweisen. Dank dieser Vertiefung, kann eine Verriegelungsschraube oder eine Verriegelungsbolzen leichter zentriert und durch den Einsatz gebohrt werden und es entstehen weniger Späne des Materials "m".

Der Einsatz kann auch mehrere Vertiefungen aufweisen, welche gleich angeordnet sind wie die Querbohrungen des Nagels.

Bei einer weiteren Ausführungsform kann der stabförmige Einsatz konisch ausgebildet sein. Dank dieser Form kann der Einsatz leichter von distal in die Längsbohrung des Nagels eingeführt werden und zudem ist eine Presspassung möglich.

Bei einer weiteren Ausführungsform wie der stabförmige Einsatz und die Wandung des Nagels zusammenwirkende Mittel auf, vorzugsweise in Form einer Nute und einer dazu passenden Erhebung, welche den Einsatz rotativ in eine vorausbestimmte Position relativ zum Nagel festlegt.

Der stabförmige Einsatz kann eine oder mehrere radial, quer zu seiner Längsachse verlaufende Erhebungen aufweisen. Diese Erhebungen können gleich angeordnet sein wie die Querbohrungen im Nagel. Die Erhebungen weisen eine Querausdehnung "x" auf, welche vorteilhafterweise im Verhältnis 1 < x/q < 2 steht, wobei "q" der Durchmesser des Einsatzes (7) ist._Der Vorteil dieser Ausführung besteht darin, dass die Erhebungen beim Einführen des Einsatzes in die Längsbohrung des Nagels in die Querbohrungen einschnappen, so dass der Einsatz eindeutig und sicher im Nagel positioniert ist. Im weiteren führt das erhöhte Verdrängungsvolumen zu einer verbesserten Haltekraft, d.h. einer erhöhten Winkelstabilität.

Der Nagel kann bereits mit einem in seine Längsbohrung bis in den Bereich der Querbohrungen eingeführten Einsatz dem Chirurgen zur Verfügung gestellt werden oder alternativ als separat verpackte Teile.

Der Nagel kann zusammen mit einer Verriegelungsschraube mit einem Schraubenschaft und einem Aussengewinde verwendet werden, wobei für den Durchmesser d des Schraubengewindes a > d < b gilt, und "d" vorzugsweise mindestens 5 % kleiner ist als die kleinere der beiden Dimensionen a,b.

Zur Herstellung des Nagels kann vom oberen oder unteren Ende des Nagels (aus dem Material M) ein Festkörper aus einem Material "m" in die Längsbohrung des Nagels eingeführt werden, so dass der Festkörper mindestens in den Bereich einer der Querbohrungen des Nagels zu liegen kommt.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 einen Längsschnitt durch einen mit einem weicheren Material partiell gefüllten hohlen Marknagel;
Fig. 2 einen Querschnitt des Marknagels im Bereich der Querbohrung;
Fig. 3 einen Einsatz aus biokompatiblem Kunststoff zur Einführung von distal in einen hohlen Marknagel mit radialen Ansenkungen, respektiven Durchbohrungen, entsprechend der Position der Querbohrungen im Marknagel;
Fig. 4 einen Querschnitt des Marknagels im Bereich der Querbohrung mit einem rotationssichernden Einsatz;
Fig. 5 eine Ansicht eines rotationssichernden Einsatzes aus biokompatiblem Kunststoff zur Einführung von distal in einen hohlen Marknagel mit radialen Erhebungen entsprechend der Position der Querbohrungen im Marknagel;
Fig. 6 einen Längsschnitt durch einen Einsatz aus biokompatiblem Kunststoff zur Einführung von proximal in einen hohlen Marknagel;
Fig. 7 einen um 90° gedrehten Längsschnitt durch den Einsatz nach Fig. 6; und
Fig. 8 eine Ansicht eines Einsatzes zur Einführung von proximal über die gesamte Länge des hohlen Marknagels.

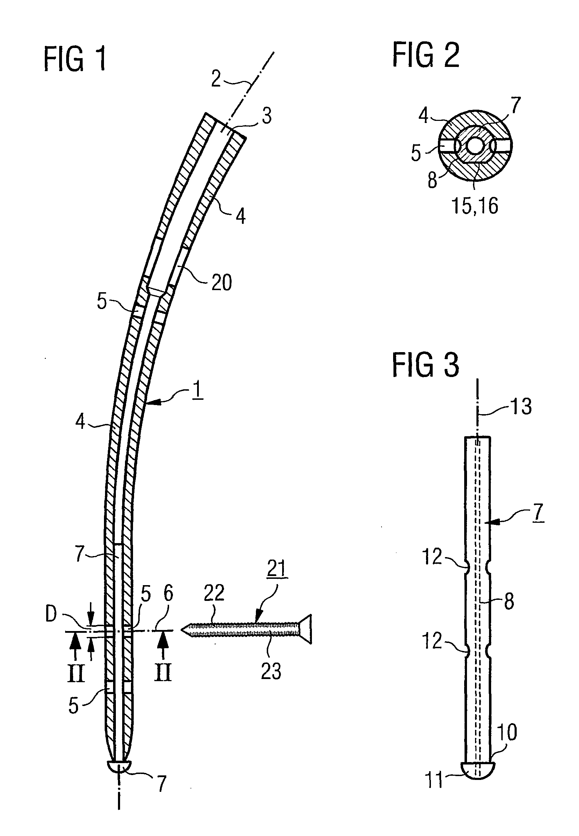

Der in Fig. 1 dargestellte chirurgische Nagel 1 ist ein intramedullärer Marknagel für Röhrenknochen mit einer Zentralachse 2 der aus einem Material M (Metall oder Metallegierung) besteht und drei quer zur Zentralachse 2 verlaufende Querbohrung 5 mit dem Durchmesser D und einer Querachse 6 aufweist.

Eine vierte Querbohrungen ist proximal angebracht und als Langloch 20 ausgebildet, wobei die längere Dimension in axialer Richtung angeordnet ist. Zwei der drei Querbohrungen 5 sind im distalen Teil des Marknagels 1 angebracht.

Der Marknagel besitzt eine koaxial zur Zentralachse 2 verlaufende Längsbohrung 3 und dadurch eine Wandung 4. In diese Längsbohrung 3 ist von distal her ein stabförmiger Einsatz 7 (Fig. 3) in Form eines einstückigen Festkörpers aus resorbierbarem Polylactid eingesetzt, so dass die Längsbohrung 3 im Bereich der beiden distalen Querbohrungen 5 mit einem Material m geringerer Festigkeit, insbesondere einem geringeren Elastizitätsmodul (im Vergleich zum Material M des Marknagels) passgenau gefüllt ist. Möglich ist aber auch eine Presspassung des Materials m.

Wie in Fig. 1 dargestellt kann eine Verriegelungsschraube 21 mit dem Schaft 22 und dem Aussengewinde 23 in die Querbohrung 5 und damit durch den Einsatz 7 eingeschraubt werden.

Der Einsatz 7 besitzt eine zu seiner Längsachse 13 koaxiale, durchgehende Längsbohrung 8 und an seinem distalen Ende eine halbkugelförmige Erweiterung 11 mit einem nach proximal gerichteten Anschlag 10. Durch den Anschlag 10 der Erweiterung 11 wird eine sichere axiale Positionierung des Einsatzes 7 in der Längsbohrung 3 des Marknagels garantiert.

Der Einsatz 7 besitzt vier radiale, quer zur Längsachse 13 verlaufende Vertiefungen 12, welche axial so angeordnet sind, dass sie auf die gleiche Höhe zu liegen kommen wie die Querbohrungen 5 des Marknagels, wenn der Einsatz 7 bis zum Anschlag 10 in die Längsbohrung 3 eingeführt ist. Damit die Vertiefungen 12 auch radial gesehen mit den Bohrungen 5 fluchten, weist der Einsatz 7, wie in Fig. 2 dargestellt ein Profil 15 auf, welches mit einem Profil 16 in der Längsbohrung 3 des Marknagels korrespondiert, so dass der Einsatz 7 nur in einer bestimmten rotativen Position in die Längsbohrung 3 einführbar ist.

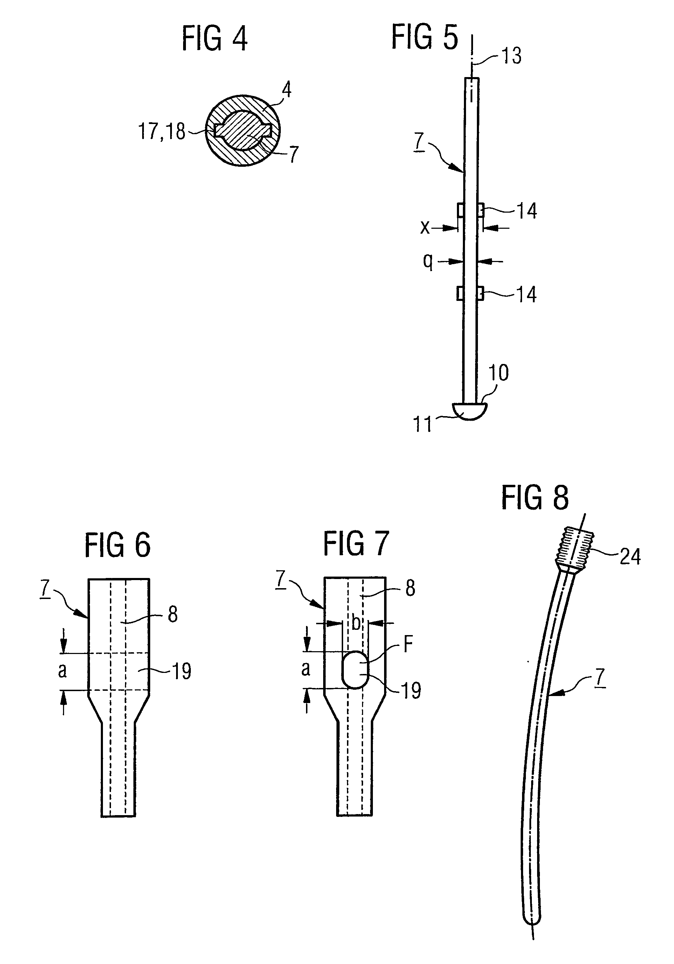

Wie in Fig. 4 dargestellt, kann der Einsatz 7 und die Längsbohrung 3 des Marknagels statt der Profile 15,16 in Form von Abflachungen auch zwei Rippen/Nuten 17,18 aufweisen, welche den gleichen Effekt der Rotationsblockierung bewirken.

In Fig. 5 ist sodann eine Alternative zum Einsatz 7 nach Fig. 3 dargestellt, bei welchem statt der Vertiefungen 12, Erhebungen 14 vorhanden sind, welche dank ihrer Elastizität in die Öffnungen der Querbohrungen 5 in der Wandung 4 einschnappen können, so dass ebenfalls eine axiale und rotative Sicherung des Einsatzes 7 erreicht werden kann. Die Querausdehnung x der Erhebungen 14 steht im Verhältnis 1 < x/q < 2, wobei q der Durchmesser des Einsatzes 7 ist.

In den Fig. 6 und 7 ist ein weiterer alternativer Einsatz 7 dargestellt, der statt von distal, von proximal in die Längsbohrung 3 des Marknagels einführbar ist. Er weist eine axiale Längsbohrung 8 auf, sowie ein zum Langloch 20 im Marknagel 1 korrespondierendes Langloch 19 auf. Das Langloch 19 ist annähernd elliptisch mit einer langen Achse a und einer kurzen Achse b.

In Fig. 8 ist eine weitere Ausführungsform eines Einsatzes 7 dargestellt, der in etwa die gleiche Länge wie der Marknagel aufweist und somit sämtliche Querbohrungen 5 (Verriegelungsbohrungen) des Marknagels von proximal bis distal abdeckt. Der Einsatz 7 wird durch ein Gewinde 24 im proximalen Bereich des Einsatzes 7 im hohlen Marknagel fixiert. Der Einsatz 7 kann bei Bedarf auch noch intraoperativ gekürzt werden.

## Patentansprüche

1. Chirurgischer Nagel (1), insbesondere intramedullärer Marknagel, mit einer Zentralachse (2), der aus einem Material M mit der Zugfestigkeit F_{z} , der Druckfestigkeit F_{d} , der Dichte ρ₂ und dem Elastizitätsmodul E besteht und mindestens eine quer zur Zentralachse (2) verlaufenden Querbohrung (5) mit dem Querschnittsprofil F und einer Querachse (6) aufweist, wobei das Querschnittsprofil F in Richtung der Zentralachse eine maximale Länge a und senkrecht dazu eine maximale Breite b aufweist, wobei der Nagel (1) eine koaxial zur Zentralachse (2) verlaufende Längsbohrung (3) und eine Wandung (4) aufweist, und wobei der Nagel (1) einen über die Längsbohrung (3) in den Bereich der Querbohrung (5) einführbaren Einsatz (7) mit der Längsachse (13) aus einem Material m umfasst, welches ein geringeres Elastizitätsmodul e < E aufweist als das Material M, wobei
C) der Einsatz (7) stabförmig ausgebildet ist, und
D) der Einsatz (7) eine zentrale Längsbohrung (8) aufweist;
**dadurch gekennzeichnet, dass**
A) der Nagel (1) mindestens zwei Querbohrungen (5) aufweist; und
B) der Einsatz (7) axial bis in den Bereich der mindestens zwei Querbohrungen (5) einführbar ist, so dass er die Querbohrungen (5) überdeckt.

2. Nagel (1) nach Anspruch 1, **dadurch gekennzeichnet, dass** der Einsatz (7) und die Wandung (4) zusammenwirkende Mittel aufweisen, welche den Einsatz (7) rotativ in eine vorausbestimmte Position relativ zum Nagel (1) festlegen.

3. Nagel (1) nach Anspruch 1 oder 2, **dadurch gekennzeichnet, dass** der Einsatz (7) Erhebungen (14) aufweist, welche in die Querbohrungen (5) in der Wandung (4) einschnappen können, so dass eine axiale und rotative Sicherung des Einsatzes (7) erreichbar ist.

4. Nagel (1) nach einem der Ansprüche 1 bis 3, **dadurch gekennzeichnet, dass** er mindestens drei Querbohrungen (5) aufweist.

5. Nagel (1) nach einem der Ansprüche 1 bis 4, **dadurch gekennzeichnet, dass** mindestens eine der Querbohrungen (5) als Langloch (20) mit dem Querschnittsprofil F ausgebildet ist, wobei die längere Dimension a in axialer Richtung angeordnet ist.

6. Nagel (1) nach einem der Ansprüche 1 bis 5, **dadurch gekennzeichnet, dass** der Durchmesser der Längsbohrung (3) in Richtung der Zentralachse (2) variabel gestaltet ist.

7. Nagel (1) nach Anspruch 6, **dadurch gekennzeichnet, dass** die Längsbohrung (3) vorzugsweise einen zirkulären Absatz aufweist.

8. Nagel (1) nach einem der Ansprüche 1 bis 7, **dadurch gekennzeichnet, dass** der Einsatz (7) mindestens eine radial, quer zur Längsachse (8) verlaufende Vertiefung (12) aufweist und dass der Einsatz (7) stabförmig ausgebildet ist.

9. Nagel (1) nach einem der Ansprüche 1 bis 8, **dadurch gekennzeichnet, dass** der Einsatz (7) konisch und stabförmig ausgebildet ist.

10. Nagel (1) nach einem der Ansprüche 2 bis 9, **dadurch gekennzeichnet, dass** die zusammenwirkenden Mittel in Form einer Nute und einer dazu passenden Erhebung realisiert sind.

11. Nagel (1) nach einem der Ansprüche 1 bis 10, **dadurch gekennzeichnet, dass** der Einsatz (7) mindestens eine radial, quer zur Längsachse (13) verlaufende Erhebung (14) aufweist und stabförmig ausgebildet ist.

12. Nagel (1) nach Anspruch 11, **dadurch gekennzeichnet, dass** der Einsatz (7) mehrere Erhebungen (14) aufweist, welche gleich angeordnet sind wie die Querbohrungen (5) im Nagel (1).

13. Nagel (1) nach Anspruch 11 oder 12, **dadurch gekennzeichnet, dass** die Erhebungen (14) eine Querausdehnung "x" aufweisen und diese im Verhältnis 1 < x/q < 2 stehen, wobei "q" der Durchmesser des Einsatzes (7) ist.

14. Ein System mit einem Nagel (1) nach einem der Ansprüche 1 bis 13 und einer in die Querbohrung (5) und durch den Einsatz (7) einführbaren Verriegelungsschraube oder einem Verriegelungsbolzen (21), **dadurch gekennzeichnet, dass** die Verriegelungsschraube oder der Verriegelungsbolzen (21) ein Aussengewinde (23), beziehungsweise einen gewindelosen Schaft (22), mit einem Aussendurchmesser "d" aufweist, welcher der Bedingung a > d < b gehorcht.

15. Ein System mit einem Nagel (1) nach Anspruch 14, **dadurch gekennzeichnet, dass** für den Durchmesser "d" des Schraubengewindes (23) die Bedingung a > d < b gilt, und "d" vorzugsweise mindestens 5 % kleiner ist als die kleinere der beiden Dimensionen a,b.

## Claims

1. Surgical pin (1), especially an intramedullary pin, with a central axis (2) and consisting of a material M with the tensile strength F_{z}, the compression strength F_{d}, the density ρ₂ and the modulus of elasticity E and having at least one transverse borehole (5), extending transversely to the central axis (2), with the cross-sectional profile F and a transverse axis (6), the cross-sectional profile F having a maximum length a in the direction of the central axis and, perpendicularly thereto, a maximum width b, the pin (1) having a longitudinal borehole (3), which extends coaxially with the central axis (2), and a wall (4), and the pin (1) comprising an insert (7), which can be introduced via the longitudinal borehole (3) into the region of the transverse borehole (5), the insert (7) having a longitudinal axis (13) and consisting of a material m, the modulus of elasticity e of which is less than the modulus of elasticity E of the material M, whereby
A) the insert (7) is configured rod-shapedly; and
B) the insert (7) has a central longitudinal borehole (8),
**characterized in that**
C) the pin (1) has at least two transverse boreholes (5); and
D) the insert (7) can be introduced axially into the region of the at least two transverse boreholes (5) so that it covers the transverse boreholes (5).

2. The pin of claim 1, **characterized in that** the insert (7) and the wall (4) have interacting means, which fix the insert (7) rotatively in a previously determined position relative to the pin (1).

3. The pin (1) of claims 1 or 2, **characterized in that** the insert (7) has elevations (14), which can snap into the transverse boreholes (5) in the wall (4), so that an axial and rotative securing of the insert (7) can be obtained.

4. The pin (1) of one of the claims 1 to 3, **characterized in that** it has at least three transverse boreholes (5).

5. The pin (1) of one of the claims 1 to 4, **characterized in that** at least one of the transverse boreholes (5) is constructed as an elongated hole (20) with the cross-sectional profile F, the longer dimension a being disposed in the axial direction.

6. The pin (1) of one of the claims 1 to 5, **characterized in that** the diameter of the longitudinal borehole (3) is configured variably in the direction of the central axis (2).

7. The pin (1) of one of the claims 1 to 6, **characterized in that** and the longitudinal borehole (3) preferably has a circular shoulder.

8. The pin (1) of one of the claims 1 to 8, **characterized in that** the insert (7) has at least one depression extending radially and transversely to the longitudinal axis (8) and that the insert (7) is configured rod-shapedly.

9. The pin (1) of one of the claims 1 to 9, **characterized in that** the insert (7) is constructed conically and rod-shapedly.

10. The pin (1) of one of the claims 2 to 9, **characterized in that** the interacting means are realized in the form of a groove and an elevation fitting thereto.

11. The pin (1) of one of the claims 1 to 10, **characterized in that** the insert (7) has at least one elevation (14), which extends radially and transversely to the longitudinal axis (13) and that the insert (7) is configured rod-shapedly.

12. The pin (1) of claim 11, **characterized in that** the insert (7) has several elevations (14), which are disposed similarly to the transverse boreholes (5) in the pin (1).

13. The pin (1) of claims 11 or 12, **characterized in that** the elevations (14) have a transverse extent "x", which fulfills the condition 1 < x/q < 2, "q" being the diameter of the insert (7).

14. A system with a pin (1) of one of the claims 1 to 13 and a locking screw or a locking bolt (21), which can be introduced into the transverse borehole (5) and through the insert (7), **characterized in that** the locking screw or locking bolt (21) is provided with an external thread (23), respectively a threadless shaft (22) which has an external diameter "d", which fulfills the condition a > d < b.

15. A system with a pin (1) of claim 14, **characterized in that** the diameter "d" of the screw thread (23) fulfills the condition a > d < b and "d" preferably being at least 5% smaller than the smaller of the two dimensions a, b.

## Revendications

1. Clou chirurgical (1), en particulier clou intramédullaire, ayant un axe central (2), fait d'un matériau M ayant une résistance à la traction F_{Z}, une résistance à la compression F_{d}, une densité ρ₂ et un module d'élasticité E, et présentant au moins un alésage transversal (5) s'étendant transversalement à l'axe central (2) et ayant un profil transversal F et un axe transversal (6), dans lequel le profil transversal F présente, dans le sens de l'axe central, une longueur maximale a et, perpendiculairement à celui-ci, une largeur maximale b, dans lequel le clou (1) présente un alésage longitudinal (3) s'étendant coaxialement à l'axe central (2) et une paroi (4), et dans lequel le clou (1) présente un insert (7), ayant un axe longitudinal (13), pouvant être inséré dans l'alésage longitudinal (3) jusque dans la zone de l'alésage transversal (5), et fait d'un matériau m, lequel matériau présente un module d'élasticité e < E inférieur à celui du matériau M, dans lequel :
- A) l'insert (7) a la forme d'un barreau ; et
- B) l'insert (7) présente un alésage longitudinal central (8) ;
**caractérisé en ce que**
- C) le clou (1) présente au moins deux alésages transversaux (5) ; et
- D) l'insert (7) peut être inséré axialement jusque dans la zone des au
moins deux alésages transversaux (5),
de sorte qu'il obture les alésages transversaux (5).

2. Clou (1) selon la revendication 1, **caractérisé en ce que** l'insert (7) et la paroi (4) présentent des moyens complémentaires qui bloquent l'insert (7) en rotation, dans une position prédéterminée par rapport au clou (1).

3. Clou (1) selon la revendication 1 ou 2, **caractérisé en ce que** l'insert (7) présente des protubérances (14) qui peuvent s'encliqueter dans les alésages transversaux (5) prévus dans la paroi (4), de sorte qu'un blocage axial et un blocage en rotation de l'insert (7) puissent être obtenus.

4. Clou (1) selon l'une quelconque des revendications 1 à 3, **caractérisé en ce qu'**il présente au moins trois alésages transversaux (5).

5. Clou (1) selon l'une quelconque des revendications 1 à 4, **caractérisé en ce qu'**au moins un des alésages transversaux (5) est réalisé sous la forme d'un trou oblong (20) de profil transversal F, dans lequel la dimension la plus longue a s'étend dans le sens axial.

6. Clou (1) selon l'une quelconque des revendications 1 à 5, **caractérisé en ce que** le diamètre de l'alésage longitudinal (3) est réalisé de manière variable dans le sens de l'axe central (2).

7. Clou (1) selon la revendication 6, **caractérisé en ce que** l'alésage longitudinal (3) présente de préférence un épaulement circulaire.

8. Clou (1) selon l'une quelconque des revendications 1 à 7, **caractérisé en ce que** l'insert (7) présente au moins un évidement (12) s'étendant radialement et transversalement à l'axe longitudinal (8) et **en ce que** l'insert (7) est réalisé sous la forme d'un barreau.

9. Clou (1) selon l'une quelconque des revendications 1 à 8, **caractérisé en ce que** l'insert (7) est réalisé sous forme conique et sous la forme d'un barreau.

10. Clou (1) selon l'une quelconque des revendications 2 à 9, **caractérisé en ce que** les moyens complémentaires sont réalisés sous la forme d'une rainure et d'une saillie correspondante.

11. Clou (1) selon l'une quelconque des revendications 1 à 10, **caractérisé en ce que** l'insert (7) présente au moins une protubérance (14) s'étendant radialement et transversalement à l'axe longitudinal (13) et **en ce que** l'insert est réalisé sous la forme d'un barreau.

12. Clou (1) selon la revendication 11, **caractérisé en ce que** l'insert (7) présente plusieurs protubérances (14) disposées de manière correspondante par rapport aux alésages transversaux (5) du clou (1).

13. Clou (1) selon la revendication 11 ou 12, **caractérisé en ce que** les protubérances (14) présentent une extension latérale « x » et **en ce que** celle-ci se trouve dans un rapport 1 < x/q < 2, où « q » représente le diamètre de l'insert (7).

14. Système comprenant un clou (1) selon l'une quelconque des revendications 1 à 13 et une vis de verrouillage ou un boulon de verrouillage (21) pouvant être inséré dans l'alésage transversal (5) et à travers l'insert (7), **caractérisé en ce que** la vis de verrouillage ou le boulon de verrouillage (21) présente un filetage (23), ou une tige non filetée (22), de diamètre extérieur « d », lequel remplit la condition a > d < b.

15. Système comprenant un clou (1) selon la revendication 14, **caractérisé en ce que** la condition a > d < b s'applique au diamètre « d » du pas de vis (23), et **en ce que** « d » est de préférence inférieur d'au moins 5% à la dimension la plus petite des deux dimensions a, b.
